# EUROPEAN PATENT APPLICATION

(11) **EP 1 813 277 A1**
(43) Date of publication of application: **01.08.2007**
(21) Application number: 07001625.8
(22) Date of filing: 25.01.2007
(51) Int. Cl.: A61K 31/70

(54) **Dispenser for dispensing two or more substances**

(30) Priority: 25.01.2006 US 339650
(71) Applicant: Imaginative Research Associates, Inc., Woburn MA 01801 (US)
(72) Inventor: Vishnupad, Mohan, New York, New York 10021 (US); Vishnupad, Naomi, Reading, Massachusetts 01867 (US)
(74) Representative: Laufhütte, Dieter

(57) **Abstract**

Two separate compositions, one containing an effective anti-acne treating amount of a first active ingredient and one containing a second active ingredient that is incompatible with the first active ingredient are packaged within and dispensed from a common dispenser. By packaging these two active ingredients in this manner, long shelflife and convenient dispensing and application are provided.

## Description

### RELATED APPLICATIONS

This application is a continuation-in-part of U.S. Application Serial No. 10/121,839 filed on April 12, 2002 which is a continuation-in-part of U.S. Application Serial No. 09/734,748 filed on December 12, 2000, now U.S. Patent No. 6,462,025. The entire disclosures of both of these priority documents are incorporated herein by this reference.

### BACKGROUND

### Technical Field

This disclosure relates to compositions and apparatus for dispensing two or more distinct substances. More specifically, this disclosure relates to compositions and apparatus which allow long-term storage and subsequent dispensing of two compositions, to wit, a first composition containing two or more active ingredients and a second composition containing an active ingredient that is incompatible with at least one of the active ingredients in the first composition.

### Background of Related Art

Acne is a common inflammatory disease of human skin, and concentrates in skin areas where sebaceous glands are largest, most numerous, and most active. In its milder types, it is a more or less superficial disorder which is evidenced by slight, spotty irritations and ordinary skin hygiene is a satisfactory treatment. However, in the more inflammatory types of acne, bacterial invasion of or about the pilosebaceous follicles occurs and pustules, infected cysts and, in extreme cases, canalizing inflamed and infected sacs appear. These lesions may become extensive and leave permanent, disfiguring scars.

Acne is very common by puberty and at least 80% of teenagers are afflicted. The facial eruptions are known to cause such psychic trauma in many adolescents that they find it difficult to make personal adjustments and consequently, withdraw and self-pity occur. The sufferer may be constantly aware of the obvious facial blemishes. For these reasons a medicinal preparation and treatment are of definite benefit and may eliminate the need for psychotherapy.

To reduce the severity of acne, various forms of medication have previously been topically applied to the skin. Antibacterial soaps have been used as well as bactericidal agents such as sulfur and resorcinol. Other topical compositions have separately contained benzoyl peroxide, hexachlorophene, erythromycin or neomycin sulfate. None of these prior preparations has been completely effective.

As disclosed by Klein et al. (U.S. Pat. No. 4,497,794), it was discovered that a mixture on the skin of a peroxide, especially benzoyl peroxide and an antibiotic or antibacterial such as clindamycin, neomycin, sodium sulfacetamide, sulfur, tetracycline or erythromycin is particularly beneficial as they can exert a statistically significant synergistic effect. Peroxides inhibit the formation of free fatty acids in the skin, primarily through inactivation of extracellular lipase (via oxidation) necessary to cleave triglycerides into free fatty acids and glycerol. The antibiotic or antibacterial component reduces the concentration of Corynebacterium acnes (i.e., P. acnes), a normal anaerobic bacteria which is the prime source of the lipase. Instead of the benzoyl peroxide, which is preferred, peroxides such as stabilized hydrogen peroxide and peroxides of organic acids, such as a lauroyl peroxide, may be used.

As disclosed by Klein et al., erythromycin and benzoyl peroxide may be applied to the skin in combination in a preformulated aqueous-alcoholic gel. However, if a mixture is first made up and then applied to the skin, it is best that the mixture be made at the time of application or that the mixture be used within twenty-four hours. The prompt use of a premix is necessary due to the chemical incompatibility of the two active agents. Because of this, it is advisable that the two agents be put in separate vials, bottles or other containers. For example, the Klein et al. patent discloses a kit containing, separately bottled liquid compositions comprising 5% benzoyl peroxide and a solution of erythromycin in ethanol or acetone.

However, separately packaging multiple dosages of the two active ingredients presents a number of disadvantages to the end-user. For example, a unit application dosage of each active must be removed sequentially from each container and absorbed onto an applicator, such as a cotton swab, so that it can be coated onto the skin of the user. This provides opportunities for spillage or over- or under-dosing, which can lead to skin irritation and other side effects. Furthermore, such a multidose system necessarily adds to the costs of packaging, shipping and storage.

A dispensing and applicator system intended to overcome these difficulties is disclosed in U.S. Patent No. 5,562,642. A dual-pad package is disclosed therein that purportedly can contain, preserve and deliver single unit doses of two or more chemically- or physically-incompatible active ingredients. For example, an antibiotic in combination with a liquid, semi-liquid (cream) or gelled aqueous or non-aqueous vehicle can be absorbed by and retained by the first pad and a second ingredient which is physically- or chemically-incompatible with the antibiotic, such as a peroxide, can be absorbed and retained by the second pad, preferably in combination with the appropriate vehicle.

It would be desirable to provide a means for simultaneously dispensing two active acne treating compounds in aesthetically acceptable vehicles which allow prolonged shelf life for both active compounds and easy mixing just prior to application to the skin.

### SUMMARY

It has now been discovered that two separate compositions, one containing a first active ingredient which is effective in treating acne and one containing a second active ingredient that is incompatible with the first active ingredient can be packaged within and dispensed from a common dispenser. More particularly, a dual dispenser and has two chambers and a pump means for removing first and second compositions from the chambers through one or more outlets. The first chamber contains a first composition that includes a first active ingredient that is effective in treating acne; and the second chamber contains a second composition containing a second active ingredient that is incompatible with the first active ingredient. Preferably, both the first and second active ingredients are effective against acne. By packaging the two active ingredients in this manner, long shelflife and convenient dispensing and application are provided.

In embodiments, the first active ingredient is an antibiotic and the second active ingredient is benzoyl peroxide. In other embodiments, the first active ingredient is an antibiotic and the second active ingredient is a retinoid . In other embodiments, the first active ingredient is benzoyl peroxide and the second active ingredient is a retinoid.

In embodiments, the present disclosure relates to an apparatus including a first chamber containing a first composition that contains a plurality of active ingredients at least one of which is effective in treating acne and a second chamber containing a second composition that includes at least one active ingredient that is incompatible with one of the plurality of active ingredients in the first composition. The apparatus also includes a pump for moving the first and second compositions out of the first and second chambers, respectively, and one or more outlets for dispensing the first and second compositions. In embodiments, the first composition contains both antibiotic and a retinoid and the second composition contains benzoyl peroxide. In other embodiments, the first chamber contains an antibiotic and benzoyl peroxide and the second composition contains a retinoid.

### BRIEF DESCRIPTION OF THE DRAWINGS

Various embodiments are described herein with reference to the drawing wherein:

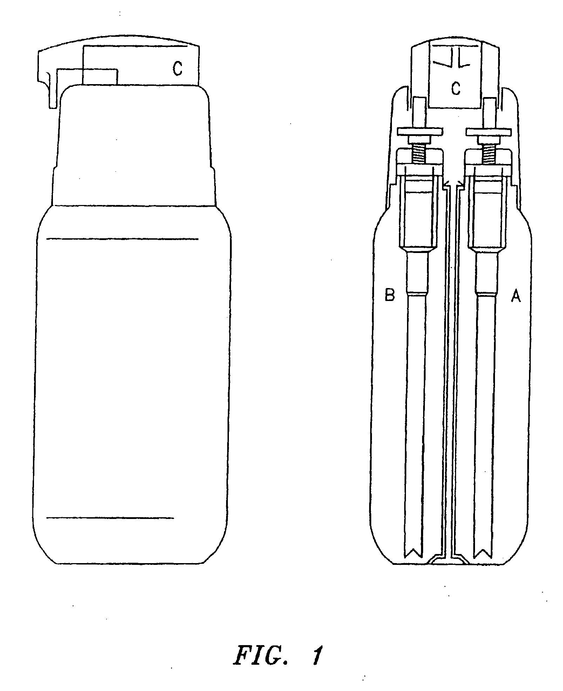

FIG. 1 is a schematic view of a container suitable for dispensing the first and second compositions in accordance with this disclosure.

### DETAILED DESCRIPTION OF PREFERRED EMBODIMENTS

The dual dispensers described herein include a first chamber containing a first composition, a second chamber containing a second composition and pump means for simultaneously dispensing the first and second compositions. The first and second compositions can be any cream, lotion, gel emulsion or suspension that is of an appropriate consistency to be pumped out of the chambers by pump means. The first and second compositions can thus have a viscosity greater than about 1000 centipoise (cps) when measured using a Brookfield viscometer (model LVT) at room temperature using spindle number 3 or 4 at 0.3 to 30 rpm. It should be understood that all viscosities referred to herein are measured in this manner. In embodiments, the first and second compositions have a viscosity greater than 5,000 cps. In embodiments, the compositions have a viscosity from about 1000 to about two million centipoise. In other embodiments, the first and second compositions have a viscosity from about 10,000 cps to about 1,000,000 cps. For purposes of presenting a composition with a good feel to the user, the first and second compositions advantageously have viscosities that differ by no greater than 25%.

The first composition contains a first active ingredient effective in treating acne. The first composition can be substantially anhydrous or aqueous. In embodiments the first composition may be substantially anhydrous. By the term "substantially anhydrous" it is meant that, other than water of hydration contained in the various components used to formulate the composition, no free water is added to the composition. Typically, the water content of the composition will be less than 5% by weight. Preferably, the water content of the composition is less than 3% and most preferably less than about 1% by weight of the composition. However, where the first active ingredient is not sensitive to water, then aqueous formulations are acceptable for the first composition, including solutions, suspensions and water-in-oil emulsions.

One class of active ingredients known to be effective in treating acne is antibiotics. Preferably, the antibiotic is one currently known to be useful in treating acne, such as, for example, erythromycin, tetracyclin, clindamycin, their derivatives or pharmaceutically acceptable salts. The antibiotic may be present in the first composition in an effective acne-treating amount. The antibiotic may be present in the first composition in an amount from about 0.001 wt. % to about 5 wt. %, in embodiments from about 0.1 wt. % to about 1.0 wt. %.

In embodiments, the first composition contains a polar solvent, a thickening agent and an antibiotic.

Polar solvents useful in the first composition include polyols. A polyol is a compound with at least two hydroxyl groups per molecule, i.e., a compound having multiple hydroxyl groups as part of its molecular structure. Among the useful polyols are polyhydric alcohols. Propylene glycol, dipropylene glycol, polyethylene glycol and glycerine are particularly preferred polar solvents for use in the first composition.

The thickening agent used in the first composition may be selected from the group consisting of acrylic acid polymers and polyacrylamides. The thickening agent can be used in an amount sufficient to obtain a composition of viscosity in the desired range.

Useful acrylic acid polymers include copolymers of (meth)acrylic acid and of monomers containing at least one fatty chain; these monomers are chosen from hydrophobic monomers with a fatty chain, amphiphilic monomers containing a hydrophobic part with a fatty chain and a hydrophilic part, or alternatively their mixtures. Suitable materials include, for example, copolymers of C₁₀₋₃₀ alkyl acrylates with one or more monomers of acrylic acid, methacrylic acid, or one of their short chain (i.e., C₁₋₄ alcohol) esters, wherein the crosslinking agent is an allyl ether of sucrose or pentaerythritol. These copolymers are commonly referred to as acrylates/C₁₀₋₃₀ alkyl acrylate crosspolymers and are commercially available under the tradename CARBOPOL® from B.F. Goodrich, Cleveland, Ohio U.S.A. Other polymers useful in the preparation of the present compositions are polymers of polyacrylic acid crosslinked with from about 0.75% to about 2.0% of polyalkyl sucrose or polyalkyl pentaerythritol often with molecular weights of 4 to 5 million or more that are commercially available, for example, under the trade designation CARBOPOL® 934, 940 and 941 from B.F. Goodrich, Cleveland, Ohio U.S.A. Anionic amphiphilic polymers which contain 95% to 60% by weight of acrylic recurring structural units, 4% to 40% by weight of acrylate recurring structural units and 0.1% to 6% by weight of crosslinking monomer, or (ii) which contain 98% to 96% by weight of acrylic recurring structural units, 1% to 4% by weight of acrylate recurring structural units and 0.1 % to 0.6% by weight of crosslinking monomer are also useful as the thickening agent in the present compositions. Such polymers include, for example, those hydrophobically-modified cross-linked polymers of acrylic acid having amphipathic properties marketed by B.F. Goodrich under the trademarks CARBOPOL® 1342 and CARBOPOL® 1382. Also useful is IULTREZ® 10 (available from B. F. Goodrich), an oil in water emulsion of a modified acrylic copolymer comprising of a major portion of a monoolefinically unsaturated carboxylic acid monomer or its anhydride having a length of from about 3 to 6 carbon atoms and a minor portion of a C₈₋₃₀ chain acrylate or methacrylate ester monomer wherein the carboxylic acid or its anhydride is from about 80 to about 99% by weight and the C₈₋₃₀ chain acrylate or methacrylate ester monomer is from about 1% to about 20% by weight. The polymer is described in U.S. Pat. No. 5,004,598, hereby incorporated by reference in its entirety.

When used, these acrylic acid polymers may be present in the first composition at a level from about 0.05% to about 20%, in embodiments from about 0.5% to 10% and in other embodiments from about 1% to about 10%.

Where the first composition is an anhydrous antibiotic composition, the first composition can alternatively contain polyacrylamide polymers as the thickening agent, especially nonionic polyacrylamide polymers. Useful non-ionic polymers are branched or unbranched polyacrylamides and substituted polyacrylamides. These polymers are non-ionic polymers which can be formed from a variety of monomers including acrylamide and methacrylamide which are unsubstituted or substituted with one or two alkyl groups (preferably C₁₋₅). Preferred acrylate amides and methacrylate amides in which the amide nitrogen is unsubstituted, or substituted with one or two C₁₋₅ alkyl groups (preferably: methyl, ethyl or propyl), for example, acrylamide, methacrylamide, N-methylacrylamide, N-methylmethacrylamide, N,N-dimethylmethacrylamide, N-isopropylacrylamide, N-isopropylmethacrylamide and N,N-dimethylacrylamide. These monomers are generally disclosed in U.S. Pat. No. 4,963,348 which is incorporated by reference herein in its entirety. These copolymers may optionally be formed using conventional neutral crosslinking agents such as dialkenyl compounds. The use of such crosslinking agents for cationic polymers is disclosed in U.S. Pat. Nos. 4,628,078 and 4,599,379 both of which are incorporated by reference herein. These non-ionic copolymers may have a molecular weight greater than about 1,000,000 preferably greater than about 1,500,000 and range up to about 30,000,000. Most preferred among these polyacrylamide polymers is the nonionic polymer given the CTFA designation polyacrylamide and isoparaffin and laureth-7, available under the tradename SEPIGEL® 305 from Seppic Corporation (Fairfield, N.J.). Other polyacrylamide polymers useful herein include multi-block copolymers of acrylamides and substituted acrylamides with acrylic acids and substituted acrylic acids. Commercially available examples of these multi-block copolymers include Hypan SR150H, SS500V, SS500W, SSSA100H, from Lipo Chemicals, Inc., (Patterson, N.J.).

When used, these non-ionic polyacrylamides may be present in the first composition at a level from about 0.05% to about 20%, in embodiments from about 0.5% to 10% and in other embodiments from about 1% to about 10%.

Quite surprisingly, it has been found that contrary to product literature relating to the commercially available acrylic acid polymers and polyacrylamides, when used to formulate the first composition as a substantially anhydrous composition, the thickening agents need not be dispersed in an aqueous medium or neutralized to provide the desired thickening.

Benzoyl peroxide is another active ingredient known to be an effective anti-acne treatment that can be incorporated into the first composition. Where benzoyl peroxide is the first active ingredient, the first composition can be either substantially anhydrous or may contain water and can be any benzoyl peroxide-containing cream, lotion, gel or suspension. Examples of benzoyl peroxide compositions that are suitable for use in accordance with this disclosure include, but are not limited to the compositions disclosed in U.S. Patent No. 5,632,996, the disclosure of which is incorporated herein by reference. In embodiments, the benzoyl peroxide composition may be substantially anhydrous. Among these embodiments are compositions containing a) a polar solvent, b) a thickening agent selected from the group consisting of acrylic acid polymers, polyacrylamides and combinations thereof (as described above), c) benzoyl peroxide, d) alkyl benzoate and, optionally e) a synthetic cleanser. Suitable synthetic cleansers include, but are not limited to sodium cocoyl isethionate, alpha olefin sulfonate sarcosynates and acyl glutamates.

The amount of benzoyl peroxide in the composition may be from about 0.1 to about 20 percent by weight based on the total weight of the composition, preferably from about 1.0 to about 15 weight percent, most preferably from about 1.5 to about 10 weight percent. The benzoyl peroxide-containing composition may have a viscosity greater than about 1000 centipoise (cps) when measured using a Brookfield viscometer (model LVT) at room temperature using spindle number 3 or 4 at 0.3 to 30 rpm, in embodiments, greater than 5,000 cps. In other embodiments, the benzoyl peroxide-containing composition has a viscosity from about 1000 to about two million centipoise. In yet other embodiments, the benzoyl peroxide-containing composition has a viscosity of about 10,000 cps to about 1,000,000 cps.

In alternative embodiments, the first composition contains a retinoid. Suitable retinoids, include, for example, retinol, retinoic acid, retinyl palmitate, retinyl propionate or retinyl acetate as well as synthetic retinoid mimics. The retinoid may be present in the second composition in an amount from about 0.001 wt. % to about 5 wt. %, in embodiments from about 0.1 wt. % to about 2.0 wt. %.

Where a retinoid is the first active ingredient, the first composition can be either substantially anhydrous or may contain water. Aqueous retinoid compositions are known to those skilled in the art and include, for example, certain compositions commercially available under the RETIN-A^{®} tradename from Johnson and Johnson, Inc., New Brunswick, New Jersey. Suitable retinoid compositions are also disclosed in U.S. Patent Nos. 4,690,825 and 5,955,109, the entire disclosures of which are incorporated herein by this reference. In embodiments, the retinoid-containing compositions may be substantially anhydrous and may contain a polar solvent, a thickening agent and a retinoid. Suitable polar solvents and thickening agents for the second composition are the same as described above for the antibiotic and benzoyl peroxide compositions described above. The retinoid-containing composition can have a viscosity greater than about 1000 centipoise (cps) when measured using a Brookfield viscometer (model LVT) at room temperature using spindle number 3 or 4 at 0.3 to 30 rpm, in embodiments, greater than 5,000 cps. In other embodiments, the retinoid-containing composition has a viscosity in the range of from about 1000 to about two million centipoise. In yet other embodiments, retinoid-containing composition has a viscosity in the range of about 10,000 cps to about 1,000,000 cps.

The second composition contains a second active ingredient that is incompatible with the first active ingredient. The second active ingredient may be effective in treating acne or may provide some other beneficial effect upon topical administration to a user's skin (such as, for example, alpha-hydroxy acids or anti-irritants The second composition can be substantially anhydrous or aqueous.

Combinations of first and second active ingredients for use in the first and second compositions include but are mot limited to: a) anibiotic in the first composition and benzoyl peroxide in the second composition; b) antibiotic in the first composition and a retinoid in the second composition; and c) benzoyl peroxide in the first composition and a retinoid in the second composition.

It should also be understood that either the first or second compositions may contain more than one active ingredient. For example, the first composition may include both antibiotic and benzoyl peroxide (such compositions being commercially available, for example, under the tradenmame DUAC^{®}, from Stiefel Laboratories, Inc., Coral Gables, FL), and the second composition may contain a retinoid. Useful compositions that include both antibiotic and benzoyl peroxide are also disclosed in U.S. Patent Nos. 5,466,446; 5,446,028; 5,767,098; and 6,013,637, the entire disclosures of which are incorporated herein by this reference. As another example, the , the first composition may include both antibiotic and retinoid (such compositions being described, for example, in commonly owned pending U.S. Patent Application Serial No. 11/198,613 filed on August 5, 2005, the entire disclosure of which is incorporated herein by this reference), and the second composition may contain benzoyl peroxide. Those skilled in the art reading this disclosure will readily envision other combinations of active ingredients useful in the first and second compositions.

The first and second compositions preferably have viscosities that are similar to provide a cosmetically elegant product when the first and second compositions are simultaneously dispensed. In embodiments, the difference in viscosity between the first and second compositions is no more than about 25%.

In addition to the above-listed ingredients, one or both of the first and second compositions may also contain a variety of non-essential ingredients such as, for example, co-solvents, preservatives, emollients, humectants, anti-inflammatory agents, antioxidants, insect repellents or skin cooling compounds, etc. For example, either of the first or second composition may contain one or more co-solvents, such as ethanol, acetone or propylene carbonate.

A preservative can also be used in either or both of the first or second compositions. Preservatives suitable for use in connection with the present compositions include parabens, sorbates, benzyl alcohol, diazolidinyl urea and isothiazolinones. Preservatives can be present in an amount from about 0.001 wt. % to about 15 wt. % of the total composition.

One or both of the first or second compositions can also be formulated to contain about 0.01 wt. % to about 30 wt. %, in embodiments about 1.0 wt. % to about 15 wt. % of the total composition, skin cooling compounds, such as menthol, methyl glycerol, asymmetrical carbonates, thiocarbonates and urethanes, substituted carboxamides, ureas or phosphine oxides as described in J. Cosmet. Chem., vol. 29, page 185 (1978) and incorporated herein by reference, methyl lactate and menthone glycerin acetal.

The first and second compositions are stored in and dispensed from a multi-chamber dispenser. Dispensing systems that include pump means suited for simultaneously dosing two separately contained incompatible compounds are well known. As such, the dispensing system schematically depicted in FIG. 1 (dispenser from Maplast, Tradate, Italy) is just one example out of a number of products which range from small, two-chambered single use pouches to tubes using different product compartments or tubes compartmentalized using extrudable, viscous and relatively inert materials to separate the incompatible compounds.

The dispenser shown in FIG. 1 is able to simultaneously dose two compounds separately contained in A and B by pressing dosing head C. Pressing dosing head C activates two small pumps which subsequently dispense the two compounds in approximately equal volumes. Depending on the design of the dosing head, the compounds can be dosed in two separate streams or in just one stream. If desired, a dispensing unit that is able to deliver The first and second substantially anhydrous compositions in a ratio, such as, for example, 1:2 can be used. Translated to the dispenser depicted in FIG. 1, this would mean that one of the two pumps is able to dose at least twice the volume of the other pump in just one stroke of dosing head C. Translated to a two-chambered single use pouch, this would mean that the chamber containing the first substantially anhydrous composition contains at least half as much product volume as the other chamber. Translated to a two-compartment tube, this would mean that under equal pressure the discharge orifice for the compartment containing the first substantially anhydrous composition allows the passage of at least twice as much product as the discharge orifice of the other compartment. Translated to a tube which is compartmentalized using extrudable material, this would mean that first substantially anhydrous composition is present inside the tube in at least double the volume of the second substantially anhydrous composition.

Other suitable dispensers are disclosed in U. S. Patent Nos. 5,356,040; 5,823,391, and 4,826,048 the disclosures of which are incorporated herein in their entirety by this reference.

The following examples are presented to illustrate specific embodiments of the present compositions and methods. These examples should not be interpreted as limitations upon the scope of the invention. All amounts are in percentage by weight based on the weight of the entire composition unless otherwise noted.

### EXAMPLES I - IV

The following formulations are exemplary of substantially anhydrous antibiotic compositions suitable for use in the apparatus described herein:

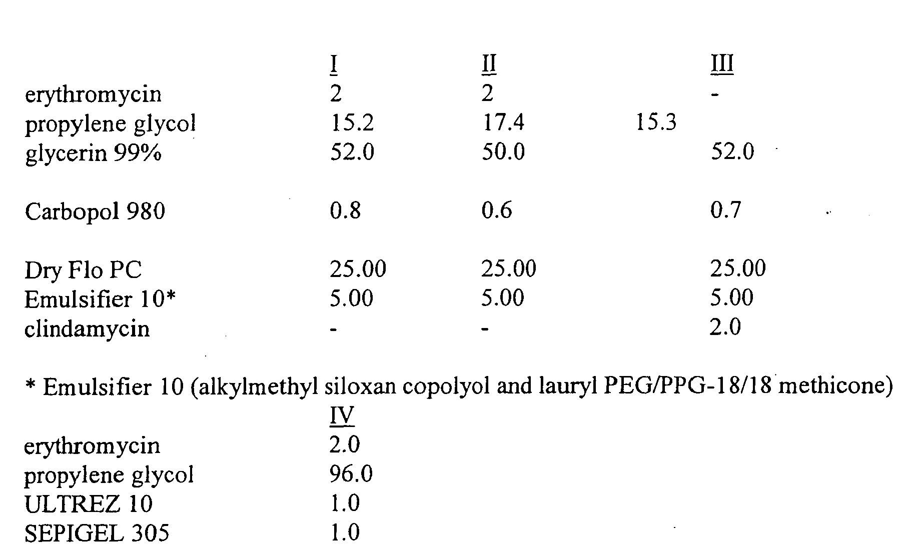

### EXAMPLES V - VII

The following formulations are exemplary of antibiotic/and or tretinoin emulsions suitable for use in the apparatus described herein:

| | V | VI | VII |
|---|---|---|---|
| Water | 78.684 | 79.83 | 76.524 |
| Carbopol 980 | 0.50 | 0.50 | 0.50 |
| Disodium EDTA | 0.50 | 0.50 | 0.50 |
| Glycerin | 2.00 | 5.00 | 2.00 |
| Sodium Hydroxide | 0.20 | 0.20 | 0.35 |
| Sorbic acid | 0.10 | 0.10 | 0.10 |
| BHT | 0.05 | - | 0.10 |
| Steareth S-20 | 1.40 | 1.40 | 1.40 |
| Steareth S-2 | 1.50 | 1.50 | 1.50 |
| Cetyl Stearyl Alcohol | 2.50 | 2.50 | 2.50 |
| Arlacel 165* | 2.00 | 2.00 | 2.00 |
| Emulsifier 10 | 1.00 | 1.00 | 1.00 |
| Fluilan (lanolin oil) | 0.47 | 0.47 | 0.47 |
| Isopropyl myristate (IPM) | 7.00 | - | 7.00 |
| Tween 20 (polysorbate 20) | 1.00 | 1.00 | 1.00 |
| Anhydrous alcohol | 1.00 | - | 1.00 |
| Tretinoin | 0.056 | - | 0.056 |
| Clindymycin | - | 2.00 | 2.00 |

| | | | |
|---|---|---|---|
| * Arlacel 165 (glyceryl stearate & PEG-100 Stearate) | | | |

### EXAMPLES VIII - XIII

The following exemplary benzoyl peroxide-containing formulations are suitable for use in the apparatus described herein to be dispensed simultaneously with any of the anhydrous formulations of Examples I-IV or the emulsion examples V-VII.

### Emulsion Compositions

| | VIII | IX |
|---|---|---|
| Water | 56.4 70.16 | |
| Carbopol 980 | - | 0.50 |
| Glycerine | 5.0 | 3.00 |
| SEPIGEL 305 | 2.0 | - |
| Sodium Hydroxide | 1.60 | 0.20 |
| Lactic acid | - | 0.10 |
| Steareth S-20 | 2.0 | 1.40 |
| Steareth S-2 | 2.0 | 1.90 |
| Cetyl Stearyl Alcohol | 3.0 | 1.90 |
| Arlacel 165* | - | 1.90 |
| Emulsifier 10 | - | 2.30 |
| Silicone Cupoydoyl | 5.0 | - |
| Fluilan (lanolin oil) | - | 0.47 |
| Lucidol 75% (Benzoyl Peroxide) | 16.0 | 6.67 |
| C₁₂₋₁₅ Benzoate Ester | 7.00 | 8.00 |
| Tween 20 (polysorbate 20) | - | 1.00 |
| Disodim EDTA | - | 0.50 |

### Benzoyl Peroxide Gels

| | X | XI |
|---|---|---|
| propylene glycol | 88.5 | 18.00 |
| Carbopol 980 | - | 0.80 |
| Glycerin | - | 42.04 |
| ULTREZ 10 | 1.5 | - |
| Dry Flo PC | - | 25.00 |
| Emulsifier 10 | - | 5.00 |
| Disodium EDTA | - | 0.15 |
| Benzoyl Peroxide | 5.0 | 6.01 |
| Fin Solv TN | 5.0 | 3.00 |

### Benzoyl Peroxide Gel Cleansers

| | XII |
|---|---|
| glycerin | 68.0 |
| ULTREZ 10 | 2.0 |
| Benzoyl Peroxide | 5.0 |
| Fin Solv TN | 5.0 |
| Sodium Cocoyl Isethionate | 20.0 |

| | XIII |
|---|---|
| Petrotalatum | 15.50 |
| Sodium Cocoyl Isethionate | 5.00 |
| Alfa olefin Sulfonate | 2.00 |
| Titaniam Dioxide | 0.30 |
| Lucidol 75% (Benzoyl Peroxide) | 15.80 |
| C₁₂₋₁₅ Alkyl Benzoate | 7.00 |
| Triethanolamine | 0.60 |
| Carbopol 1382 | 0.80 |
| Glycerin | 53.0 |

### EXAMPLES XIV - XV

The following exemplary formulations containing two actives are suitable for use in the apparatus described herein:

### Emulsion Formulations (Active combination: clindamycin/tretinoin)

| Ingredient | XIV | XV |
|---|---|---|
| Dionized water | 71.5626 | 75.6824 |
| Disodium EDTA | 0.4 | 0.5 |
| NaOH (10%) | - | 0.35 |
| Carbopol 980 | 0.20 | 0.65 |
| Steareth S-2 | 1.216 | 1.9 |
| Stearate (and) PEG-100 stearate | 0.896 | 2.5 |
| Cetyl stearyl alcohol | 1.216 | 3.0 |
| Emulsifier 10 | 0.800 | 2.3 |
| Tween 20 | 0.80 | - |
| Fluilan | - | 0.36 |
| Glycerin | 13.79 | 1.9 |
| Butyl hydroxyl toluene | 0.040 | 0.05 |
| Stearaths-21 | - | 1.40 |
| Sorbic acid | 0.08 | 0.10 |
| Clindamycin phosphate (100% active) | 1.00 | 1.255 |
| Tretinoic acid | 0.0504 | 0.526 |
| Ethyl alcohol, anhydrous | 0.80 | 1.0 |
| Alkyl benzoate | 7.149 | - |
| IPM | - | 7.0 |

### BPO/Clindamycin Examples

### Emulsion Compositions

| | VIII | IX |
|---|---|---|
| Water | 54.4 | 68.16 |
| Carbopol 980 | - | 0.50 |
| Glycerine | 5.0 | 3.00 |
| SEPIGEL 305 | 2.0 | - |
| Sodium Hydroxide | 1.60 | 0.20 |
| Lactic acid | - | 0.10 |
| Steareth S-20 | 2.0 | 1.40 |
| Steareth S-2 | 2.0 | 1.90 |
| Cetyl Stearyl Alcohol | 3.0 | 1.90 |
| Arlacel 165* | - | 1.90 |
| Emulsifier 10 | - | 2.30 |
| Silicone Cupoydoyl | 5.0 | - |
| Fluilan (lanolin oil) | - | 0.47 |
| Lucidol 75% (Benzoyl Peroxide) | 16.0 | 6.67 |
| C₁₂₋₁₅ Benzoate Ester | 7.00 | 8.00 |
| Tween 20 (polysorbate 20) | - | 1.00 |
| Disodim EDTA | - | 0.50 |
| Clindamycin phosphate (100%) | 2.00 | 2.00 |

### Gel Compositions

| | X | XI |
|---|---|---|
| propylene glycol | 86.5 | 16.00 |
| Carbopol 980 | - | 0.80 |
| Glycerin | - | 42.04 |
| ULTREZ 10 | 1.5 | - |
| Dry Flo PC | - | 25.00 |
| Emulsifier 10 | - | 5.00 |
| Disodium EDTA | - | 0:15 |
| Benzoyl Peroxide | 5.0 | 6.01 |
| Fin Solv TN | 5.0 | 3.00 |
| Clindamycin Phosphate (100%) | 2.00 | 2.00 |

It will be understood that various modifications may be made to the embodiments disclosed herein. Therefore, the above description should not be construed as limiting, but merely as exemplifications of preferred embodiments. Those skilled in art will envision other modifications within the scope and spirit of the claims appended hereto.

## Claims

1. An apparatus comprising:
a first chamber containing a first composition, the first composition comprising a plurality of active ingredients at least one of which is effective in treating acne;
a second chamber containing a second composition comprising at least one active ingredient that is incompatible with one of the plurality of active ingredients in the first compositon;
pump means for moving the first and second compositions out of the first and second chambers, respectively; and
one or more outlets for dispensing the first and second compositions.

2. An apparatus as in claim 1 wherein the first composition comprises one or more antibiotics and one or more retinoids.

3. An apparatus as in claim 1 wherein the first composition comprises one or more antibiotics and benzoyl peroxide.

4. An apparatus as in claim 2 wherein the one or more antibiotics are selected from the group consisting of erythromycin, clindamycin, tetracycline, derivatives of erythromycin, clindamycin or tetracycline and pharmaceutically acceptable salts of erythromycin, clindamycin or tetracycline.

5. An apparatus as in claim 3 wherein the one or more antibiotic are selected from the group consisting of erythromycin, clindamycin, tetracycline, derivatives of erythromycin, clindamycin or tetracycline and pharmaceutically acceptable salts of erythromycin, clindamycin or tetracycline.

6. An apparatus as in claim 2 wherein the one or more retinoids are selected from the group consisting of retinol, retinoic acid, retinyl palmitate, retinyl propionate, retinyl acetate and synthetic retinoid mimetics.

7. An apparatus comprising:
a first chamber containing a first composition, the first composition comprising an antibiotic and a retinoid;
a second chamber containing a second composition comprising benzoyl peroxide;
pump means for moving the first and second compositions out of the first and second chambers, respectively; and
one or more outlets for dispensing the first and second compositions.

8. An apparatus as in claim 7 wherein the antibiotic is selected from the group consisting of erythromycin, clindamycin, tetracycline, derivatives of erythromycin, clindamycin or tetracycline and pharmaceutically acceptable salts of erythromycin, clindamycin or tetracycline.

9. An apparatus as in claim 7 wherein the retinoid is selected from the group consisting of retinol, retinoic acid, retinyl palmitate, retinyl propionate, retinyl acetate and synthetic retinoid mimetics.

10. An apparatus comprising:
a first chamber containing a first composition, the first composition comprising an antibiotic and benzoyl peroxide;
a second chamber containing a second composition comprising a retinoid;
pump means for moving the first and second compositions out of the first and second chambers, respectively; and
one or more outlets for dispensing the first and second compositions.

11. An apparatus as in claim 10 wherein the retinoid is selected from the group consisting of retinol, retinoic acid, retinyl palmitate, retinyl propionate, retinyl acetate and synthetic retinoid mimetics.

12. An apparatus as in claim 10 wherein the antibiotic is selected from the group consisting of erythromycin, clindamycin, tetracycline, derivatives of erythromycin, clindamycin or tetracycline and pharmaceutically acceptable salts of erythromycin, clindamycin or tetracycline.

13. A method of treating acne comprising
simultaneously pumping a first composition and a second composition from first and second chambers, respectively,
the first composition comprising an antibiotic and a retinoidt, the second composition comprising benzoyl peroxide; and
contacting the first composition and second composition with the skin of a person afflicted with acne.

14. A method as in claim 13 wherein the antibiotic is selected from the group consisting of erythromycin, clindamycin, tetracycline, derivatives of erythromycin, clindamycin or tetracycline and pharmaceutically acceptable salts of erythromycin, clindamycin or tetracycline.

15. A method as in claim 13 wherein the retinoid is selected from the group consisting of retinol, retinoic acid, retinyl palmitate, retinyl propionate, retinyl acetate and synthetic retinoid mimetics.

16. A method of treating acne comprising
simultaneously pumping a first composition and a second composition from first and second chambers, respectively,
the first composition comprising an antibiotic and benzoyl peroxide, the second composition comprising a retinoid; and
contacting the first composition and second composition with the skin of a person afflicted with acne.

17. A method as in claim 16 wherein the antibiotic is selected from the group consisting of erythromycin, clindamycin, tetracycline, derivatives of erythromycin, clindamycin or tetracycline and pharmaceutically acceptable salts of erythromycin, clindamycin or tetracycline.

18. A method as in claim 16 wherein benzoyl peroxide comprises from about 0.1 to about 25 weight percent of the second composition.

19. A method as in claim 16 wherein the retinoid is selected from the group consisting of retinol, retinoic acid, retinyl palmitate, retinyl propionate, retinyl acetate and synthetic retinoid mimetics.
